# EUROPEAN PATENT SPECIFICATION

(11) **EP 0 547 359 B1**
(45) Date of publication and mention of the grant of the patent: **27.03.2002**
(21) Application number: 92118965.0
(22) Date of filing: 05.11.1992
(51) Int. Cl.: C12N 15/54, C12N 9/12, C12Q 1/68, C12N 1/21

(54) **Purified thermostable dna polymerase obtainable from pyrococcus species**
Gereinigte thermostabile DNS-Polymerase aus Pyrococcus-Arten
DNA polymérase thermostable purifiée à obtenir des espèces de pyrococcus

(30) Priority: 18.12.1991 US 809822
(43) Date of publication of application: 23.06.1993
(73) Proprietor: NEW ENGLAND BIOLABS, INC., Beverly Massachusetts 01915 (US)
(72) Inventor: Comb, Donald G., Beverly, MA 01915 (US); Kucera, Rebecca, Beverly, MA 01915 (US); Perler, Francine, Brookline, MA 02146 (US); Kong, Huimin, Beverly, MA 01915 (US); Jack, William E., Wenham, MA 01984 (US)
(74) Representative: Davies, Jonathan Mark

(56) References cited:
- EP-A- 0 258 017
- EP-A- 0 455 430
- WO-A-92/09689
- NUCLEIC ACIDS RESEARCH. vol. 19, no. 24, 1991, LONDON, GB page 6952 E.J. MATHUR ET AL. 'The DNA polymerase gene from the hyperthermophilic marine archaebacterium Pyrococcus-furiosus shows sequence homology with alpha-like DNA polymerases'
- GENE vol. 108, no. 1, 1991, AMSTERDAM, NL pages 1 - 6 K.S. LUNDBERG ET AL. 'High-fidelity amplification using a thermostable DNA-polymerase isolated from Pyrococcus furiosus'
- BIOTECHNOLOGY ABSTRACTS. DERWENT PUBLICATIONS LTD. LONDON, GB. ABSTRACT NO. 91-07714 K.S. LUNDBERG ET AL. 'A new thermostable polymerase with high fidelity thermostable DNA-polymerase isolated from Pyrococcus furiosus' & FASEB J. 1991, vol. 5, no. 6, page A1549
- BIOTECHNOLOGY ABSTRACTS. DERWENT PUBLICATIONS LTD. LONDON, GB. ABSTRACT NO. 92-02293 E.J. MATHUR ET AL. 'Characterization of a DNA-polymerase isolated from the extremely thermophilic Archaebacterium, Pyrococcus furiosus - Thermostable enzyme application in high fidelity DNA synthesis' & J. CELL BIOL. 1991, vol. 115, no. 3, Pt. 2, page 80A
- ABSTR. ANNU. MEET. AM. SOC. MICROBIOL. , US 90 MEET., 1990 page 214 J.S. TANG ET AL. 'Use of a set of degenerate oligonucleotide primers for amplification of the polymerase I genes from thermophilic and mesophilic isolates'
- JOURNAL OF CHEMICAL TECHNOLOGY AND BIOTECHNOLOGY vol. 42, no. 4, 1988, pages 315 - 317 K.O. STETTER 'Hyperthermophiles physiology and enzymes - potential source of thermostable beta-galactosidase, RNA-polymerase, amylases, proteases, etc....'

### SEQUENCE LISTING

(1) GENERAL INFORMATION:
   (i) APPLICANT:
      COMB, DONALD G.
      PERLER, FRANCINE
      KUCERA, REBECCA
      KONG, HUIMIN
      JACK, WILLIAM F.
   (ii) TITLE OF INVENTION: PURIFIED THERMOSTABLE DNA POLYMERASE OBTAINABLE FROM PYROCOCCUS SPECIES
   (iii) NUMBER OF SEQUENCES: 2
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
(2) INFORMATION FOR SEQ ID NO:1:
   (i) SEQUENCE CHARACTERISTICS:
      (A) LENGTH: 3420 base pairs
      (B) TYPE: nucleic acid
      (C) STRANDEDNESS: double
      (D) TOPOLOGY: not relevant
   (xi) SEQUENCE DESCRIPTION: SEQ ID NO:1:
   GGATCCCTCT CTTTTTGGTA ACCCCATACG TCATTCCCTC AACCAAAACT TCAGCATCGT   60
   TGCAGTGGTC AGTGTGTCTG TGGGAGATGA AGAGGACGTC GATTTTTCTG GGGTCTATCT  120
   TGTATCTCCA CATTCTAACT AACGCTCCAG GCCCAGGATC AACGTAGATG TTTTTGCTCG  180
   CCTTAATGAA GAAGCCACCA GTGGCTCTTG CCTGCGTTAT CGTGACGAAC CTTCCACCAC  240
   CGCCACCGAG AAAAGTTATC TCTATCATCT CACACCTCCC CCATAACATC ACCTGCTCAA  300
   TTTTTAAGCG TTCTTAAAGG CTTAAATACG TGAATTTAGC GTAAATTATT GAGGGATTAA  360
   GTATGATACT TGACGCTGAC TACATCACCG AGGATGGGAA GCCGATTATA AGGATTTTCA  420
   AGAAAGAAAA CGGCGAGTTT AAGGTTGAGT ACGACAGAAA CTTTAGACCT TACATTTACG  480
   CTCTCCTCAA AGATGACTCG CAGATTGATG AGGTTAGGAA GATAACCGCC GAGAGGCATG  540
   GGAAGATAGT GAGAATTATA GATGCCGAAA AGGTAAGGAA GAAGTTCCTG GGGAGGCCGA  600
   TTGAGGTATG GAGGCTGTAC TTTGAACACC CTCAGGACGT TCCCGCAATA AGGGATAAGA  660
   TAAGAGAGCA TTCCGCAGTT ATTGACATCT TTGAGTACGA CATTCCGTTC GCGAAGAGGT  720
   ACCTAATAGA CAAAGGCCTA ATTCCAATGG AAGGCGATGA AGAGCTCAAG TTGCTCGCAT  780
   TTGACATAGA AACCCTCTAT CACGAAGGGG AGGAGTTCGC GAAGGGGCCC ATTATAATGA  840
   TAAGCTATGC TGATGAGGAA GAAGCCAAAG TCATAACGTG GAAAAAGATC GATCTCCCGT  900
   ACGTCGAGGT AGTTTCCAGC GAGAGGGAGA TGATAAAGCG GTTCCTCAAG GTGATAAGGG  960
   AGAAAGATCC CGATGTTATA ATTACCTACA ACGGCGATTC TTTCGACCTT CCCTATCTAG 1020
   TTAAGAGGGC CGAAAAGCTC GGGATAAAGC TACCCCTGGG AAGGGACGGT AGTGAGCCAA 1080
   AGATGCAGAG GCTTGGGGAT ATGACAGCGG TGGAGATAAA GGGAAGGATA CACTTTGACC 1140
   TCTACCACGT GATTAGGAGA ACGATAAACC TCCCAACATA CACCCTCGAG GCAGTTTATG 1200
   AGGCAATCTT CGGAAAGCCA AAGGAGAAAG TTTACGCTCA CGAGATAGCT GAGGCCTGGG 1260
   AGACTGGAAA GGGACTGGAG AGAGTTGCAA AGTATTCAAT GGAGGATGCA AAGGTAACGT 1320
   ACGAGCTCGG TAGGGAGTTC TTCCCAATGG AGGCCCAGCT TTCAAGGTTA GTCGGCCAGC 1380
   CCCTGTGGGA TGTTTCTAGG TCTTCAACTG GCAACTTGGT GGAGTGGTAC CTCCTCAGGA 1440
   AGGCCTACGA GAGGAATGAA TTGGCTCCAA ACAAGCCGGA TGAGAGGGAG TACGAGAGAA 1500
   GGCTAAGGGA GAGCTACGCT GGGGGATACG TTAAGGAGCC GGAGAAAGGG CTCTGGGAGG 1560
   GGTTAGTTTC CCTAGATTTC AGGAGCCTGT ACCCCTCGAT AATAATCACC CATAACGTCT 1620
   CACCGGATAC GCTGAACAGG GAAGGGTGTA GGGAATACGA TGTCGCCCCA GAGGTTGGGC 1680
   ACAAGTTCTG CAAGGACTTC CCGGGGTTTA TCCCCAGCCT GCTCAAGAGG TTATTGGATG 1740
   AAAGGCAAGA AATAAAAAGG AAGATGAAAG CTTCTAAAGA CCCAATCGAG AAGAAGATGC 1800
   TTGATTACAG GCAACGGGCA ATCAAAATCC TGGCAAACAG CATTTTACCG GAAGAATGGG 1860
   TTCCACTAAT TAAAAACGGT AAAGTTAAGA TATTCCGCAT TGGGGACTTC GTTGATGGAC 1920
   TTATGAAGGC GAACCAAGGA AAAGTGAAGA AAACGGGGGA TACAGAAGTT TTAGAAGTTG 1980
   CAGGAATTCA TGCGTTTTCC TTTGACAGGA AGTCCAAGAA GGCCCGTGTA ATGGCAGTGA 2040
   AAGCCGTGAT AAGACACCGT TATTCCGGAA ATGTTTATAG AATAGTCTTA AACTCTGGTA 2100
   GAAAAATAAC AATAACAGAA GGGCATAGCC TATTTGTCTA TAGGAACGGG GATCTCGTTG 2160
   AGGCAACTGG GGAGGATGTC AAAATTGGGG ATCTTCTTGC AGTTCCAAGA TCAGTAAACC 2220
   TACCAGAGAA AAGGGAACGC TTGAATATTG TTGAACTTCT TCTGAATCTC TCACCGGAAG 2280
   AGACAGAAGA TATAATACTT ACGATTCCAG TTAAAGGCAG AAAGAACTTC TTCAAGGGAA 2340
   TGTTGAGAAC ATTACGTTGG ATTTTTGGTG AGGAAAAGAG AGTAAGGACA GCGAGCCGCT 2400
   ATCTAAGACA CCTTGAAAAT CTCGGATACA TAAGGTTGAG GAAAATTGGA TACGACATCA 2460
   TTGATAAGGA GGGGCTTGAG AAATATAGAA CGTTGTACGA GAAACTTGTT GATGTTGTCC 2520
   GCTATAATGG CAACAAGAGA GAGTATTTAG TTGAATTTAA TGCTGTCCGG GACGTTATCT 2580
   CACTAATGCC AGAGGAAGAA CTGAAGGAAT GGCGTATTGG AACTAGAAAT GGATTCAGAA 2640
   TGGGTACGTT CGTAGATATT GATGAAGATT TTGCCAAGCT TCTTGGCTAC TATGTGAGCG 2700
   AGGGAAGTGC GAGGAAGTGG AAGAATCAAA CTGGAGGTTG GAGTTACACT GTGAGATTGT 2760
   ACAACGAGAA CGATGAAGTT CTTGACGACA TGGAACACTT AGCCAAGAAG TTTTTTGGGA 2820
   AAGTCAAACG TGGAAAGAAC TATGTTGAGA TACCAAAGAA AATGGCTTAT ATCATCTTTG 2880
   AGAGCCTTTG TGGGACTTTG GCAGAAAACA AAAGGGTTCC TGAGGTAATC TTTACCTCAT 2940
   CAAAGGGCGT TAGATGGGCC TTCCTTGAGG GTTATTTCAT CGGCGATGGC GATGTTCACC 3000
   CAAGCAAGAG GGTTCGCCTA TCAACGAAGA GCGAGCTTTT AGTAAATGGC CTTGTTCTCC 3060
   TACTTAACTC CCTTGGAGTA TCTGCCATTA AGCTTGGATA CGATAGCGGA GTCTACAGGG 3120
   TTTATGTAAA CGAGGAACTT AAGTTTACGG AATACAGAAA GAAAAAGAAT GTATATCACT 3180
   CTCACATTGT TCCAAAGGAT ATTCTCAAAG AAACTTTTGG TAAGGTCTTC CAGAAAAATA 3240
   TAAGTTACAA GAAATTTAGA GAGCTTGTAG AAAATGGAAA ACTTGACAGG GAGAAAGCCA 3300
   AACGCATTGA GTGGTTACTT AACGGAGATA TAGTCCTAGA TAGAGTCGTA GAGATTAAGA 3360
   GAGAGTACTA TGATGGTTAC GTTTACGATC TAAGTGTCGA TGAAGATGAG AATTTCCTTG 3420
(2) INFORMATION FOR SEQ ID NO:2:
   (i) SEQUENCE CHARACTERISTICS:
      (A) LENGTH: 5837 base pairs
      (B) TYPE: nucleic acid
      (C) STRANDEDNESS:
      (D) TOPOLOGY: not relevant
   (xi) SEQUENCE DESCRIPTION: SEQ ID NO:2:
   GAATTCGCGA TAAAATCTAT TTTCTTCCTC CATTTTTCAA TTTCAAAAAC GTAAGCATGA 60
   GCCAAACCTC TCGCCCTTTC TCTGTCCTTC CCGCTAACCC TCTTGAAAAC TCTCTCCAAA 120
   GCATTTTTTG ATGAAAGCTC ACGCTCCTCT ATGAGGGTCA GTATATCTGC AATGAGTTCG 180
   TGAAGGGTTA TTCTGTAGAA CAACTCCATG ATTTTCGATT TGGATGGGGG TTTAAAAATT 240
   TGGCGGAACT TTTATTTAAT TTGAACTCCA GTTTATATCT GGTGGTATTT ATGATACTGG 300
   ACACTGATTA CATAACAAAA GATGGCAAGC CTATAATCCG AATTTTTAAG AAAGAGAACG 360
   GGGAGTTTAA AATAGAACTT GACCCTCATT TTCAGCCCTA TATATATGCT CTTCTCAAAG 420
   ATGACTCCGC TATTGAGGAG ATAAAGGCAA TAAAGGGCGA GAGACATGGA AAAACTGTGA 480
   GAGTGCTCGA TGCAGTGAAA GTCAGGAAAA AATTTTTGGG AAGGGAAGTT GAAGTCTGGA 540
   AGCTCATTTT CGAGCATCCC CAAGACGTTC CAGCTATGCG GGGCAAAATA AGGGAACATC 600
   CAGCTGTGGT TGACATTTAC GAATATGACA TACCCTTTGC CAAGCGTTAT CTCATAGACA 660
   AGGGCTTGAT TCCCATGGAG GGAGACGAGG AGCTTAAGCT CCTTGCCTTT GATATTGAAA 720
   CGTTTTATCA TGAGGGAGAT GAATTTGGAA AGGGCGAGAT AATAATGATT AGTTATGCCG 780
   ATGAAGAAGA GGCCAGAGTA ATCACATGGA AAAATATCGA TTTGCCGTAT GTCGATGTTG 840
   TGTCCAATGA AAGAGAAATG ATAAAGCGTT TTGTTCAAGT TGTTAAAGAA AAAGACCCCG 900
   ATGTGATAAT AACTTACAAT GGGGACAATT TTGATTTGCC GTATCTCATA AAACGGGCAG 960
   AAAAGCTGGG AGTTCGGCTT GTCTTAGGAA GGGACAAAGA ACATCCCGAA CCCAAGATTC 1020
   AGAGGATGGG TGATAGTTTT GCTGTGGAAA TCAAGGGTAG AATCCACTTT GATCTTTTCC 1080
   CAGTTGTGCG AAGGACGATA AACCTCCCAA CGTATACGCT TGAGGCAGTT TATGAAGCAG 1140
   TTTTAGGAAA AACCAAAAGC AAATTAGGAG CAGAGGAAAT TGCCGCTATA TGGGAAACAG 1200
   AAGAAAGCAT GAAAAAACTA GCCCAGTACT CAATGGAAGA TGCTAGGGCA ACGTATGAGC 1260
   TCGGGAAGGA ATTCTTCCCC ATGGAAGCTG AGCTGGCAAA GCTGATAGGT CAAAGTGTAT 1320
   GGGACGTCTC GAGATCAAGC ACCGGCAACC TCGTGGAGTG GTATCTTTTA AGGGTGGCAT 1380
   ACGCGAGGAA TGAACTTGCA CCGAACAAAC CTGATGAGGA AGAGTATAAA CGGCGCTTAA 1440
   GAACAACTTA CCTGGGAGGA TATGTAAAAG AGCCAGAAAA AGGTTTGTGG GAAAATATCA 1500
   TTTATTTGGA TTTCCGCAGT CTGTACCCTT CAATAATAGT TACTCACAAC GTATCCCCAG 1560
   ATACCCTTGA AAAAGAGGGC TGTAAGAATT ACGATGTTGC TCCGATAGTA GGATATAGGT 1620
   TCTGCAAGGA CTTTCCGGGC TTTATTCCCT CCATACTCGG GGACTTAATT GCAATGAGGC 1680
   AAGATATAAA GAAGAAAATG AAATCCACAA TTGACCCGAT CGAAAAGAAA ATGCTCGATT 1740
   ATAGGCAAAG GGCTATTAAA TTGCTTGCAA ACAGCATCTT ACCCAACGAG TGGTTACCAA 1800
   TAATTGAAAA TGGAGAAATA AAATTCGTGA AAATTGGCGA GTTTATAAAC TCTTACATGG 1860
   AAAAACAGAA GGAAAACGTT AAAACAGTAG AGAATACTGA AGTTCTCGAA GTAAACAACC 1920
   TTTTTGCATT CTCATTCAAC AAAAAAATCA AAGAAAGTGA AGTCAAAAAA GTCAAAGCCC 1980
   TCATAAGACA TAAGTATAAA GGGAAAGCTT ATGAGATTCA GCTTAGCTCT GGTAGAAAAA 2040
   TTAACATAAC TGCTGGCCAT AGTCTGTTTA CAGTTAGAAA TGGAGAAATA AAGGAAGTTT 2100
   CTGGAGATGG GATAAAAGAA GGTGACCTTA TTGTAGCACC AAAGAAAATT AAACTCAATG 2160
   AAAAAGGGGT AAGCATAAAC ATTCCCGAGT TAATCTCAGA TCTTTCCGAG GAAGAAACAG 2220
   CCGACATTGT GATGACGATT TCAGCCAAGG GCAGAAAGAA CTTCTTTAAA GGAATGCTGA 2280
   GAACTTTAAG GTGGATGTTT GGAGAAGAAA ATAGAAGGAT AAGAACATTT AATCGCTATT 2340
   TGTTCCATCT CGAAAAACTA GGCCTTATCA AACTACTGCC CCGCGGATAT GAAGTTACTG 2400
   ACTGGGAGAG ATTAAAGAAA TATAAACAAC TTTACGAGAA GCTTGCTGGA AGCGTTAAGT 2460
   ACAACGGAAA CAAGAGAGAG TATTTAGTAA TGTTCAACGA GATCAAGGAT TTTATATCTT 2520
   ACTTCCCACA AAAAGAGCTC GAAGAATGGA AAATTGGAAC TCTCAATGGC TTTAGAACGA 2580
   ATTGTATTCT CAAAGTCGAT GAGGATTTTG GGAAGCTCCT AGGTTACTAT GTTAGTGAGG 2640
   GCTATGCAGG TGCACAAAAA AATAAAACTG GTGGTATCAG TTATTCGGTG AAGCTTTACA 2700
   ATGAGGACCC TAATGTTCTT GAGAGCATGA AAAATGTTGC AGAAAAATTC TTTGGCAAGG 2760
   TTAGAGTTGA CAGAAATTGC GTAAGTATAT CAAAGAAGAT GGCATACTTA GTTATGAAAT 2820
   GCCTCTGTGG AGCATTAGCC GAAAACAAGA GAATTCCTTC TGTTATACTC ACCTCTCCCG 2880
   AACCGGTACG GTGGTCATTT TTAGAGGCGT ATTTTACAGG CGATGGAGAT ATACATCCAT 2940
   CAAAAAGGTT TAGGCTCTCA ACAAAAAGCG AGCTCCTTGC AAATCAGCTT GTGTTCTTGC 3000
   TGAACTCTTT GGGAATATCC TCTGTAAAGA TAGGCTTTGA CAGTGGGGTC TATAGAGTGT 3060
   ATATAAATGA AGACCTGCAA TTTCCACAAA CGTCTAGGGA GAAAAACACA TACTACTCTA 3120
   ACTTAATTCC CAAAGAGATC CTTAGGGACG TGTTTGGAAA AGAGTTCCAA AAGAACATGA 3180
   CGTTCAAGAA ATTTAAAGAG CTTGTTGACT CTGGAAAACT TAACAGGGAG AAAGCCAAGC 3240
   TCTTGGAGTT CTTCATTAAT GGAGATATTG TCCTTGACAG AGTCAAAAGT GTTAAAGAAA 3300
   AGGACTATGA AGGGTATGTC TATGACCTAA GCGTTGAGGA TAACGAGAAC TTTCTTGTTG 3360
   GTTTTGGTTT GCTCTATGCT CACAACAGCT ATTACGGCTA TATGGGGTAT CCTAAGGCAA 3420
   GATGGTACTC GAAGGAATGT GCTGAAAGCG TTACCGCATG GGGGAGACAC TACATAGAGA 3480
   TGACGATAAG AGAAATAGAG GAAAAGTTCG GCTTTAAGGT TCTTTATGCG GACAGTGTCT 3540
   CAGGAGAAAG TGAGATCATA ATAAGGCAAA ACGGAAAGAT TAGATTTGTG AAAATAAAGG 3600
   ATCTTTTCTC TAAGGTGGAC TACAGCATTG GCGAAAAAGA ATACTGCATT CTCGAAGGTG 3660
   TTGAAGCACT AACTCTGGAC GATGACGGAA AGCTTGTCTG GAAGCCCGTC CCCTACGTGA 3720
   TGAGGCACAG AGCGAATAAA AGAATGTTCC GCATCTGGCT GACCAACAGC TGGTATATAG 3780
   ATGTTACTGA GGATCATTCT CTCATAGGCT ATCTAAACAC GTCAAAAACG AAAACTGCCA 3840
   AAAAAATCGG GGAAAGACTA AAGGAAGTAA AGCCTTTTGA ATTAGGCAAA GCAGTAAAAT 3900
   CGCTCATATG CCCAAATGCA CCGTTAAAGG ATGAGAATAC CAAAACTAGC GAAATAGCAG 3960
   TAAAATTCTG GGAGCTCGTA GGATTGATTG TAGGAGATGG AAACTGGGGT GGAGATTCTC 4020
   GTTGGGCAGA GTATTATCTT GGACTTTCAA CAGGCAAAGA TGCAGAAGAG ATAAAGCAAA 4080
   AACTTCTGGA ACCCCTAAAA ACTTATGGAG TAATCTCAAA CTATTACCCA AAAAACGAGA 4140
   AAGGGGACTT CAACATCTTG GCAAAGAGCC TTGTAAAGTT TATGAAAAGG CACTTTAAGG 4200
   ACGAAAAAGG AAGACGAAAA ATTCCAGAGT TCATGTATGA GCTTCCGGTT ACTTACATAG 4260
   AGGCATTTCT ACGAGGACTG TTTTCAGCTG ATGGTACTGT AACTATCAGG AAGGGAGTTC 4320
   CAGAGATCAG GCTAACAAAC ATTGATGCTG ACTTTCTAAG GGAAGTAAGG AAGCTTCTGT 4380
   GGATTGTTGG AATTTCAAAT TCAATATTTG CTGAGACTAC TCCAAATCGC TACAATGGTG 4440
   TTTCTACTGG AACCTACTCA AAGCATCTAA GGATCAAAAA TAAGTGGCGT TTTGCTGAAA 4500
   GGATAGGCTT TTTAATCGAG AGAAAGCAGA AGAGACTTTT AGAACATTTA AAATCAGCGA 4560
   GGGTAAAAAG GAATACCATA GATTTTGGCT TTGATCTTGT GCATGTGAAA AAAGTCGAAG 4620
   AGATACCATA CGAGGGTTAC GTTTATGACA TTGAAGTCGA AGAGACGCAT AGGTTCTTTG 4680
   CAAACAACAT CCTGGTACAC AATACTGACG GCTTTTATGC CACAATACCC GGGGAAAAGC 4740
   CTGAACTCAT TAAAAAGAAA GCCAAGGAAT TCCTAAACTA CATAAACTCC AAACTTCCAG 4800
   GTCTGCTTGA GCTTGAGTAT GAGGGCTTTT ACTTGAGAGG ATTCTTTGTT ACAAAAAAGC 4860
   GCTATGCAGT CATAGATGAA GAGGGCAGGA TAACAACAAG GGGCTTGGAA GTAGTAAGGA 4920
   GAGATTGGAG TGAGATAGCT AAGGAGACTC AGGCAAAGGT TTTAGAGGCT ATACTTAAAG 4980
   AGGGAAGTGT TGAAAAAGCT GTAGAAGTTG TTAGAGATGT TGTAGAGAAA ATAGCAAAAT 5040
   ACAGGGTTCC ACTTGAAAAG CTTGTTATCC ATGAGCAGAT TACCAGGGAT TTAAAGGACT 5100
   ACAAAGCCAT TGGCCCTCAT GTCGCGATAG CAAAAAGACT TGCCGCAAGA GGGATAAAAG 5160
   TGAAACCGGG CACAATAATA AGCTATATCG TTCTCAAAGG GAGCGGAAAG ATAAGCGATA 5220
   GGGTAATTTT ACTTACAGAA TACGATCCTA GAAAACACAA GTACGATCCG GACTACTACA 5280
   TAGAAAACCA AGTTTTGCCG GCAGTACTTA GGATACTCGA AGCGTTTGGA TACAGAAAGG 5340
   AGGATTTAAG GTATCAAAGC TCAAAACAAA CCGGCTTAGA TGCATGGCTC AAGAGGTAGC 5400
   TCTGTTGCTT TTTAGTCCAA GTTTCTCCGC GAGTCTCTCT ATCTCTCTTT TGTATTCTGC 5460
   TATGTGGTTT TCATTCACTA TTAAGTAGTC CGCCAAAGCC ATAACGCTTC CAATTCCAAA 5520
   CTTGAGCTCT TTCCAGTCTC TGGCCTCAAA TTCACTCCAT GTTTTTGGAT CGTCGCTTCT 5580
   CCCTCTTCTG CTAAGCCTCT CGAATCTTTT TCTTGGCGAA GAGTGTACAG CTATGATGAT 5640
   TATCTCTTCC TCTGGAAACG CATCTTTAAA CGTCTGAATT TCATCTAGAG ACCTCACTCC 5700
   GTCGATTATA ACTGCCTTGT ACTTCTTTAG TAGTTCTTTT ACCTTTGGGA TCGTTAATTT 5760
   TGCCACGGCA TTGTCCCCAA GCTCCTGCCT AAGCTGAATG CTCACACTGT TCATACCTTC 5820
   GGGAGTTCTT GGGATCC 5837

## Description

### FIELD OF THE INVENTION

The present invention relates to an extremely thermostable enzyme. More specifically, it relates to a thermostable DNA polymerase obtainable from *Pyrococcus sp.*, an isolated DNA sequence coding for the polymerase, vectors and microbial hosts transformed with the DNA, a process for preparation of the polymerase and a process for increasing expression of the polymerase from a host.

### BACKGROUND OF THE INVENTION

DNA polymerases are a family of enzymes involved in DNA repair and replication. Extensive research has been conducted on the isolation of DNA polymerases from mesophilic microorganisms such as *E. coli.* See, for example, Bessman, et al., *J. Biol. Chem.* (1957) 233:171-177 and Buttin and Kornberg *J. Biol. Chem.* (1966) 241:5419-5427.

Examples of DNA polymerases isolated from *E. coli* include *E. coli* DNA polymerase I, Klenow fragment of *E. coli* DNA polymerase I and T4 DNA polymerase. These enzymes have a variety of uses in recombinant DNA technology including, for example, labelling of DNA by nick translation, second-strand cDNA synthesis in cDNA cloning, and DNA sequencing. See Maniatis, et al., *Molecular Cloning: A Laboratory Manual* (1982).

Recently, U.S. Patent Nos. 4,683,195, 4,683,202 and 4,800,159 disclosed the use of the above enzymes in a process for amplifying, detecting, and/or cloning nucleic acid sequences. This process, commonly referred to as polymerase chain reaction (PCR), involves the use of a polymerase, two primers and nucleotide triphosphates to amplify existing nucleic acid sequences.

Some of the DNA polymerases discussed above possess a 3'-5' exonuclease activity which provides a proofreading function that gives DNA replication much higher fidelity than it would have if synthesis were the result of only a one base-pairing selection step. Brutlag, D. and Kornberg, A., *J. Biol. Chem.* (1972) 247:241-248. DNA polymerases with 3'-5' proofreading exonuclease activity have a substantially lower base incorporation error rate when compared with a DNA polymerase without proofreading activity. Chang, L.M.S., *J. Biol. Chem.* (1977) 252:1873-1880.

Research has also been conducted on the isolation and purification of DNA polymerases from thermophiles, such as *Thermus aquaticus.* Chien, A., et al. *J. Bacteriol*. (1976) 127:1550-1557, discloses the isolation and purification of a DNA polymerase with a temperature optimum of 80°C from *T. aquaticus* YT1 strain. The Chien, et al., purification procedure involves a four-step process. These steps involve preparation of crude extract, DEAE-Sephadex chromatography, phosphocellulose chromatography, and chromatography on DNA cellulose. Kaledin, et al., *Biokhymiyay* (1980) 45:644-651 also discloses the isolation and purification of a DNA polymerase from cells of *T. aquaticus* YT1 strain. The Kaledin, et al. purification procedure involves a six-step process. These steps involve isolation of crude extract, ammonium sulfate precipitation, DEAE-cellulose chromatography, fractionation on hydroxyapatite, fractionation on DEAE-cellulose, and chromatography on single-strand DNA-cellulose.

United States Patent No. 4,889,818 discloses a purified thermostable DNA polymerase from *T. aquaticus,* Taq polymerase, having a molecular weight of about 86,000 to 90,000 daltons prepared by a process substantially identical to the process of Kaledin with the addition of the substitution of a phosphocellulose chromatography step in lieu of chromatography on single-strand DNA-cellulose. In addition, European Patent Application 0258017 discloses Taq polymerase as the preferred enzyme for use in the PCR process discussed above.

Research has indicated that while the Taq DNA polymerase has a 5'-3' polymerase-dependent exonuclease function, the Taq DNA polymerase does not possess a 3'-5' proofreading exonuclease function. Lawyer, F.C., et al. *J. Biol. Chem.* (1989) 264:11, p. 6427-6437. Bernad, A., et al. *Cell* (1989) 59:219. As a result, Taq DNA polymerase is prone to base incorporation errors, making its use in certain applications undesirable. For example, attempting to clone an amplified gene is problematic since any one copy of the gene may contain an error due to a random misincorporation event. Depending on where in the replication cycle that error occurs (e.g., in an early replication cycle), the entire DNA amplified could contain the erroneously incorporated base, thus, giving rise to a mutated gene product. Furthermore, research has indicated that Taq DNA polymerase has a thermal stability of not more than several minutes at 100°C. Therefore, a DNA polymerase with much higher thermal stability and an associated 3' to 5' exonuclease proofreading activity is urgently needed by the scientific community. One such enzyme, the DNA polymerase from *Thermococcus litoralis*, an archaebacterium that grows at temperatures close to 100°C near submarine thermal vents, has recently been isolated and cloned in *E. coli*.

However, there is still a desire in the art to obtain and produce a purified DNA polymerase having 3'-5' proofreading exonuclease activity with even greater thermostability than DNA polymerases currently available. The availability of such an enzyme would improve the DNA polymerase processes described above. In addition, it would be useful if such a DNA polymerase were produced by recombinant DNA techniques. This would allow for the production of highly pure commercial quantities of the polymerase.

### SUMMARY OF THE INVENTION

In accordance with the present invention, there is provided a purified thermostable enzyme obtainable from *Pyrococcus sp.* which catalyzes the polymerization of DNA wherein said enzyme is obtainable from *E.coli* NEB #720 (ATCC # 68723). The thermostable enzyme obtainable from *Pyrococcus sp.,* an archaebacterium isolated from a submarine thermal vent at 2010 meters, is a DNA polymerase which has an apparent molecular weight of about 92,000-97,000 daltons, a half-life of about 8 hours at 100°C and a half-life of 23 hours at 95°C.

The DNA encoding the 92,000-97,000 daltons thermostable DNA polymerase obtainable from *Pyrococcus sp.* has been isolated and provides a means to obtain the thermostable enzyme of the present invention.

The *Pyrococcus sp.* DNA polymerase possesses 3'-5' proofreading exonuclease activity. In accordance with the present invention, it has been found that the 3'-5' proofreading activity of the Pyrococcus sp. DNA polymerase is 2.5 times that of the DNA polymerase obtainable from *T. litoralis.* As a result, *Pyrococcus sp.* DNA polymerase should have a much higher fidelity than a thermostable polymerase with no 3'-5' proofreading exonuclease function, such as Taq polymerase, and may have a greater proofreading activity than *T. litoralis* DNA polymerase. In addition, the *Pyrococcus sp.* DNA polymerase has a substantially greater thermal stability or half life at temperatures from 96°C to 103°C than the Taq polymerase.

### BRIEF DESCRIPTION OF DRAWINGS

FIG. 1A - is the ethidium bromide stained agarose gel of *Pyrococcus sp.* DNA cut with EcoR I (lane 3), BamH I (lane 4) and Hind III (lane 5). Lane 1 is λDNA cut with Hind III as markers and lane 2 is pBR322 as a marker.

FIG. 1B - is an autoradiography of a Southern Hybridization of the same gel in 1A. The ³²P-DNA probe was prepared from a 1.3 Kb Eco RI fragment that encodes the amino terminal portion of the *Thermococcus litoralis* DNA polymerase. Note that the BamH I cut *Pyrococcus sp.* DNA gives a single band of about 4-5 Kb with the probe. The fact that the 23 Kd band of Hind III cut λDNA shows up on the film is due to nonspecific hybridization to the large amount of DNA present in that band. The fact that the plasmid pBR322 lights up is due to homologous sequences in the probe.

FIG. 2 - is a restriction site map of the 4.8 Kb BamH I fragment containing the gene encoding the *Pyrococcus sp.* DNA polymerase in the pUC19 plasmid of *E*. *coli* 2207 (NEB#720).

FIG. 3 - is a photograph of the Coomassie Blue stained gel from which the approximate molecular weight of the *Pyrococcus sp.* DNA polymerase was determined. The arrow beside lane 1 indicates the polymerase band. Lane 2 contains the indicated molecular weight standards.

FIG. 4 - is a photograph of the western blot comparing *Thermococcus litoralis* DNA polymerase with *Pyrococcus sp.* DNA polymerase. Lane 1, Protein standards; Lane 2, 50 µg of a crude extract of *Thermococcus litoralis*; Lane 3, 2.0 µg of purified recombinant *Pyrococcus sp.* DNA polymerase. The arrow indicates the position of the polymerase I.

FIG. 5 - is a comparison of the polymerizing and exonuclease function of *Thermococcus litoralis* DNA polymerase and the *Pyrococcus sp.* DNA polymerase.

FIG. 6 - shows the heat stability at 95°C (O) and 100°C (O) of the recombinant *Pyrococcus sp.* DNA polymerase.

### DETAILED DESCRIPTION OF THE PREFERRED EMBODIMENTS

The present invention relates to a thermostable enzyme which is a DNA polymerase obtainable from *Pyrococcus sp.* strain GB-D. *Pyrococcus sp.* was isolated from a submarine thermal vent in the Sea of Cortez at a depth of 2,010 meters by Holger Jannasch using the deep sea submersible submarine Alvin of the Woods Hole Oceanographic Institute. A sample of *Pyrococcus sp.* strain GB-D (Archaebacterium, NEB#732) was deposited with the American Type Culture Collection on October 1, 1991, under the terms of the Budapest Treaty, and received ATCC Accession No. 55239.

This organism, *Pyrococcus sp.,* is an extremely thermophilic, sulfur metabolizing, archaebacterium, with a growth range between 65°C and 103°C.

For recovering the native protein, *Pyrococcus sp.* may be grown using any suitable technique, such as the technique described by Belkin, et al., *Arch. Microbiol.* (1985) 142:181-186, Briefly, the cells are grown in the media described in Belkin, et al., *supra,* containing 10 mg/ml of sulfur and 0.01 M Cysteine in 15 ml screw cap tubes at 95°C for 2 days. When larger amounts of cells are required, 1 liter screw cap bottles are used, and after sterilization, are inoculated with a fresh 10 ml culture and grown at 90°C-95°C for 2 days.

After cell growth, one preferred method for isolation and purification of the enzyme is accomplished using the multi-step process as follows: First, the cells, if frozen, are thawed, suspended in a suitable buffer such as buffer A (10 mM KP04 buffer, pH 7.4; 1.0 mM EDTA, 1.0 mM beta-mercaptoethanol), sonicated and centrifuged. The supernatant is then passed through a column which has a high affinity for proteins that bind to nucleic acids such as Affigel blue column (Biorad). The nucleic acids present in supernatant solution of *Pyrococcus sp.* and many of the proteins pass through the column and are thereby removed by washing the column with several column volumes of low salt buffer at pH of about 7.0. After washing, the enzyme is eluted with a linear gradient such as 0.1 to 2.0 M NaCl buffer A. The peak DNA polymerase activity is dialyzed and applied to phosphocellulose column. The column is washed and the enzyme activity eluted with a linear gradient such as 0.1 to 1.0 M NaCl in buffer A. The peak DNA polymerase activity is dialyzed and applied to an HPLC mono-S column (cation exchanger). The enzyme is eluted with a linear gradient such as 0.05 to 1.0 M NaCl in buffer A. The enzyme is about 50% pure at this stage.

The apparent molecular weight of the DNA polymerase obtainable from *Pyrococcus sp.* is between about 92,000 to 97,000 daltons when compared with protein standards of known molecular weight, such as phosphorylase B assigned a molecular weight of 97,400 daltons. It should be understood, however, that as a protein from an extreme thermophile, *Pyrococcus sp.* DNA polymerase may electrophorese at an aberrant relative molecular weight due to failure to completely denature or other instrinsic properties. The exact molecular weight of the thermostable enzyme of the present invention may be determined from the coding sequence of the *Pyrococcus sp.* DNA polymerase gene. The molecular weight of the eluted product may be determined by any technique, for example, by SDS-polyacrylamide gel electrophoresis (SDS-PAGE) using protein molecular weight markers.

Polymerase activity is preferably measured by the incorporation of radioactively labeled deoxynucleotides into DNAse-treated, or activated, DNA; following subsequent separation of the unincorporated deoxynucleotides from the DNA substrate, polymerase activity is proportional to the amount of radioactivity in the acid-insoluble fraction comprising the DNA. Lehman, I.R., et al., *J. Biol. Chem.* (1958) 233:163.

The half-life of the DNA polymerase of the present invention at 100°C is about 8 hours and about 23 hours at 95°C. The thermal stability or half-life of the DNA polymerase can be determined by preincubating the enzyme at the temperature of interest in the presence of the reaction buffer containing BSA. At predetermined time intervals, ranging up to 12 hours, small aliquots are removed, and added to reaction buffer containing DNA substrate, dNTPs, and BSA and assayed for polymerase activity using the method described above.

The thermostable enzyme of this invention may be produced by recombinant DNA techniques, as the gene encoding this enzyme has been cloned from *Pyrococcus sp.* genomic DNA. A partial DNA sequence of this gene is set forth in the Sequence Listing as SEQ ID NO:1. The complete coding sequence for the *Pyrococcus sp.* DNA polymerase can be derived from an approximately 5 Kb BamHI restriction fragment in pUC19 in *E. coli*, NEB#720. This *E. coli* strain was deposited with the American Type Culture Collection (ATCC) on October 1, 1991, under the terms of the Budapest Treaty and received Accession No. ATCC 68723.

### Cloning of the Pyrococcus sp. DNA Polymerase

The production of a recombinant form of the *Pyrococcus sp.* DNA polymerase generally includes the following steps: DNA is isolated which encodes the active form of the polymerase, either in its native form or as a fusion with other sequences which may or may not be cleaved away from the native form of the polymerase and which may or may not effect polymerase activity. Next, the gene may or may not be operably linked to appropriate control sequences for expression in either prokaryotic or eukaryotic host/vector systems. The vector preferably encodes all functions required for transformation and maintenance in a suitable host, and may encode selectable markers and/or control sequences for *Pyrococcus sp.* polymerase expression. The vector is used to transform a suitable host. Active recombinant thermostable polymerase can be produced by transformed host cultures either continuously or after induction of expression. Active thermostable polymerase can be recovered either from within host cells or from the culture media if the protein is secreted through the cell membrane.

While each of the above steps can be accomplished in a number of ways, it has been found that for cloning the DNA encoding *Pyrococcus sp.* DNA polymerase, expression of the polymerase from its own control sequences in *E. coli* results in high levels of gene expression.

It has been discovered that at least one intervening sequence is present within the *Pyrococcus* DNA polymerase gene. By comparison of the *Pyrococcus species* polymerase DNA and protein sequences of the present invention with the *Thermococcus litoralis* DNA polymerase DNA and protein, it was determined that the *Pyrococcus* intervening sequence is within the conserved pol α motif region III, and begins at nucleotide 1839 of SEQ ID NO:1.

### Cloning Vectors

Vectors useful in practicing the present invention should provide varying degrees of controlled expression of *Pyrococcus sp.* polymerase by providing some or all of the following control features: (1) promoters or sites of initiation of transcription, either directly adjacent to the start of the polymerase or as fusion proteins, (2) operators which could be used to turn gene expression on or off, (3) ribosome binding sites for improved translation, and (4) transcription or translation termination sites for improved stability. Appropriate vectors used in cloning and expression of *Pyrococcus sp.* polymerase include, for example, phage and plasmids. Example of phage include λgt11 (Promega), λDASH (Stratagene) λZapII (Stratagene). Examples of plasmids include pUC19, pBR322, pBluescript (Stratagene), pSP73 (Promega), pGW7 (ATCC No. 40166), pET3A (Rosenberg, et al., Gene, (1987) 56:125-135), and pET11C (Methods in Enzymology (1990) 185:60-89).

### Transformation and Infection

Standard protocols exist for transformation, phage infection and cell culture. Maniatis, et al., *Molecular Cloning*: *A Laboratory Manual* (1982). Of the numerous E. coli strains which can be used for plasmid transformation, the preferred strains include JM101 (ATCC No. 33876), XL1 (Stratagene), RRI (ATCC No. 31343), and BL21(DE3) plysS (*Methods in Enzymology* (1990), *supra*). *E. coli* strain XL1, ER1578 and ER1458 (Raleigh, et al., *N.A. Research* (1988) 16:1563-1575) are among the strains that can be used for λphage, and Y1089 can be used for λgt11 lysogeny. When preparing transient lysogens in Y1089 (Arasu, et al., *Experimental Parasitology* (1987) 64:281-289), a culture is infected with λgt11 recombinant phage either by a single large dose of phage or by co-culturing with a lytic host. The infected Y1089 cells are preferably grown at 37°C in the presence of the inducer IPTG resulting in buildup of recombinant protein within the lysis-defective host/phage system.

### Construction of Genomic DNA Library and Screening for Thermostable Polymerase

Probing a *Pyrococcus* genomic DNA library by cross-hybridization with radioactive probes prepared from the DNA polymerase gene of *Thermococcus litoralis* allowed for the identification and isolation of the *Pyrococcus sp.* DNA polymerase gene.

*Pyrococcus sp.* DNA can be isolated using the method described by Maniatis, et al., *Molecular Cloning: A Laboratory Manual* (1982).

The *Pyrococcus sp.* DNA once isolated can be used to construct genomic libraries as either random fragments or restriction enzyme fragments. The latter approach is preferred. Preferably, BamH I partials are prepared from *Pyrococcus sp.* genomic DNA using standard DNA restriction techniques such as described in Maniatis, et al., *supra.* Other restriction enzymes such as Sal I and XbaI can also be used.

Although methods are available to screen both plasmids and phage using antibodies or DNA probes (Young and Davis, *PNAS* (1983) 80:1194-1198; Maniatis et al., *supra*) it has been found that phage systems tend to work better and are therefore preferred for the first libraries.

Genomic libraries can be screened using the colony or plaque hybridization procedure (Maniatis, et al., *supra*) or using the antibody plaque reactivity (Young and Davis, *supra*). In the colony or plaque hybridization procedure, probes are formed by standard methods of labeling, for example, random priming or nick translation of a polymerase gene from a related organism, for example, *T. litoralis*. Maniatis, et al., *supra.* The genomic library is hybridized with labeled probe under conditions which depend on the stringency desired.

When genomic expression libraries are screened using the antibody/plaque procedure, since it is uncertain whether *Pyrococcus sp.* control regions function in *E. coli*, phage vectors which supply all necessary expression control regions such as λgt11 and λZap II are preferred for antibody screening. By cloning *Pyrococcus sp.* DNA into the BamH I site of λDASH or the EcoR I site of λgt11, *Pyrococcus sp.* polymerase may be expressed either as a fusion protein with beta-galactosidase in λgt11 or from its own endogenous promoter.

Once formed, the expression libraries can be screened with anti-*Pyrococcus sp.* DNA polymerase antiserum or if not available, by antibody against the DNA polymerase of a closely related organism (i.e. *Thermococcus litoralis*, another extreme thermophile) using standard antibody plaque hybridization procedures such as those described by Young and David, *PNAS* (1983), *supra.*

Using either procedure, the *Pyrococcus sp.* DNA polymerase DNA, coding for part or the whole gene, once identified can then be subcloned in, for example, pBR322, pBluescript, M13 or pUC19. If desired, the DNA sequence can be determined by, for example, the Sanger dideoxy chain-terminating method (Sanger, F., Nicklen, S. & Coulson, A.R. *PNAS* (1977) 74:5463-5467).

### Identification of DNA Encoding and Expression of the Pyrococcus Species DNA Polymerase.

Several methods exist for determining that the DNA sequence coding for the *Pyrococcus sp.* DNA polymerase has been obtained. These include, for example, comparing the actual or deduced amino-terminal sequence of the protein encoded by the recombinant DNA to the native protein, or determining whether the recombinant DNA produces a protein which binds antibody specific for native *Pyrococcus sp.* DNA polymerase. In addition, research by Wang, et al., *FASEB Journal* (1989) 3:14-21 suggests that certain regions of DNA polymerase sequences from the polymerase α family are highly conserved among many species as well as a separate group of conserved regions in Poll-like polymerases. As a result, by comparing the predicted amino acid sequence of the cloned gene with the amino acid sequence of known DNA polymerases, such as human DNA polymerase α and *E. coli* DNA polymerase I, the identification of these islands of homology provides strong evidence that the recombinant DNA indeed encodes a DNA polymerase.

As noted above, it has been found that DNA coding for *Pyrococcus sp.* DNA polymerase contains an intron or intervening sequence within the pol α motif region III. Therefore, as one means to increase overexpression in host cells such as *E*. *coli*, the DNA sequence coding for the intron can be deleted. There are a number of approaches known in the art which can be used to delete DNA sequences and therefore splice out an intron *in-vitro.* One method involves identifying unique restriction enzyme sites in the coding region which are near the splice junction or area to be deleted. A duplex oligomer is synthesized to bridge the gap between the 2 restriction fragments. A 3-part ligation consisting of the amino end restriction fragment, the bridging oligo and the carboxy end restriction fragment yields an intact gene with the intron deleted.

Another method is a modification of the above-described method. The majority of the intron is deleted by cutting with restriction enzymes with unique sites within the intron, but close to the coding sequence border. The linear plasmid containing a deletion of the majority of the intron is ligated together. Single strand phage are generated from the pBluescript vector recombinant by superinfection with the f1 helper phage IR1. A single strand oligomer is synthesized with the desired final sequence and is annealed to the partially deleted intron phage DNA. The remainder of the intron is thus looped out. By producing the original phage in *E. coli* strain CJ236 the Kunkel method of mutagenesis (*Methods in Enzymology,* 154:367 (1987)) can be used to select for the full deleted intron constructs.

Yet another method which can be used to delete the intron uses DNA amplification. See, for example, Maniatis, et al., *Molecular Cloning: A Laboratory Manual,* (1989), Vol. 2, 2nd edition. Briefly, primers are generated to amplify and subsequently join the amino and carboxyl halves of the gene.

When an intron is deleted *in-vitro,* using the methods discussed above, the native splice junction may be unknown. Accordingly, one skilled in the art would predict that several possible artificial splice junctions exist that would result in the production of an active enzyme.

Once identified, the DNA sequence coding for the *Pyrococcus sp.* DNA polymerase, with or without the intron deleted, can be cloned into an appropriate expression vector such as a plasmid derived from *E. coli*, for example, pET3A, pBluescript or pUC19, the plasmids derived from the *Bacillus subtilis* such as pUB110, pTP5 and pC194, plasmids derived from yeast such as pSH19 and pSH15, bacteriophage such as λphage, bacteria such as *Agrobacterium tumefaciens,* animal viruses such as retroviruses and insect viruses such as Baculovirus.

The recombinant vector is introduced into the appropriate host using standard techniques for transformation and phage infection. For example, the calcium chloride method, as described by Cohen, S.N., *PNAS* (1972) 69:2110 is used for *E. coli*. The transformation of *Bacillus* is carried out according to the method of Chang, S., et al., *Molecular and General Genetics* (1979) 168:111. Transformation of yeast is carried out according to the method of Parent, et al., *Yeast* (1985) 1:83-138. Certain plant cells can be transformed with *Agrobacterium tumefaciens,* according to the method described by Shaw, C.H., et al., *Gene* (1983) 23:315. Transformation of animal cells is carried out according to, for example, the method described in *Virology* (1973) 52:456. Transformation of insect cells with Baculovirus is carried out according to, for example, the method described in *Biotechnology* (1988) 6:47.

The transformants are cultivated, depending on the host cell used, using standard techniques appropriate to such cells. For example, for cultivating *E. coli*, cells are grown in LB media (Maniatis, *supra*) at 30°C to 42°C to mid log or stationary phase.

The *Pyrococcus sp.* DNA polymerase can be isolated and purified from a culture of transformed host cells, for example, by either extraction from cultured cells or the culture solution.

When the *Pyrococcus sp.* DNA polymerase is to be extracted from a cultured cell, the cells are collected after cultivation by methods known in the art, for example, centrifugation. Then, the collected cells are suspended in an appropriate buffer solution and disrupted by ultrasonic treatment, lysozyme and/or freeze-thawing. A crude extract containing the *Pyrococcus sp.* DNA polymerase is obtained by centrifugation and/or filtration.

When the *Pyrococcus sp.* DNA polymerase is secreted into the culture solution, i.e., alone or as a fusion protein with a secreted protein such as maltose binding protein, the supernatant is separated from the cells by methods known in the art, for example, centrifugation.

The separation and purification of the *Pyrococcus sp.* DNA polymerase contained in the culture supernatant or the cell extract can be performed by the method described above, or by appropriate combinations of known separating and purifying methods. These methods include, for example, methods utilizing solubility such as salt precipitation and solvent precipitation, methods utilizing the difference in molecular weight such as dialysis; ultra-filtration, gel-filtration, and SDS-polyacrylamide gel electrophoresis, methods utilizing a difference in electric charge such as ion-exchange column chromatography, methods utilizing specific affinity such as affinity chromatography, methods utilizing a difference in hydrophobicity such as reverse-phase high performance liquid chromatography and methods utilizing a difference in isoelectric point such as isoelectric focusing electrophoresis.

One preferred method for isolating and purification of the recombinant enzyme is accomplished using the multi-stage process as follows: First, the cells, if frozen are thawed, suspended in a suitable buffer such as Buffer A (100 mM NaCl, 25 mM Tris pH 7.5, 0.1 mM EDTA, 10% glycerol, 0.05% Triton X-100), lysed and centrifuged. The clarified crude extract is then heated to 75°C for approximately 30 minutes. The denatured proteins are removed by centrifugation. The supernatant is then passed through a column that has high affinity for proteins that bind to nucleic acids such as Affigel Blue column (Biorad). The nucleic acids present in the supernatant solution and many of proteins pass through the column and are thereby removed by washing the column with several column volumes with low-salt buffer at pH of about 7.0. After washing, the enzyme is eluted with a linear gradient such as 0.1 M to 1.5 M NaCl Buffer A. The active fractions are pooled, dialyzed and applied to a phosphocellulose column. The column is washed and DNA polymerase activity eluted with a linear gradient of 0.1 to 1.0 M NaCl in Buffer B (100 M NaCl, 15 mM KP04, 0.1 mM EDTA, 10% glycerol, 0.05% Triton X-100, pH 6.8). The fractions are collected and BSA is added to each fraction. The fractions with DNA polymerase activity are pooled. The *Pyrococcus sp.* DNA polymerase obtained may be further purified using the standard product purification techniques discussed above.

### Stabilization and Use of the Pyrococcus species DNA Polymerase.

For long-term storage, the thermostable enzyme of the present invention is stored in the following buffer: 0.05 M NaCl, 0.01 M KP04 (pH 7.4), 0.1 mM EDTA and 50% glycerol at -20°C.

The *Pyrococcus sp.* DNA polymerase of the present invention may be used for any purpose in which such an enzyme is necessary or desirable. For example, in recombinant DNA technology including, second-strand cDNA synthesis in cDNA cloning, and DNA sequencing. See Maniatis, et al., *supra.*

The *Pyrococcus sp.* DNA polymerase of the present invention may be modified chemically or genetically to inactivate the 3'-5' exonuclease function and used for any purpose in which such a modified enzyme is desirable, e.g., DNA sequencing.

For example, genetically modified *Pyrococcus sp.* DNA polymerase may be isolated by randomly mutagenizing the *Pyrococcus sp.* DNA polymerase gene and then screening for those mutants that have lost exonuclease activity, without loss of polymerase activity. Alternatively, genetically modified *Pyrococcus sp.* DNA polymerase is preferably isolated using the site-directed mutagenesis technique described in Kunkel, T.A., *PNAS* (1985) 82:488-492.

In addition, the *Pyrococcus sp.* DNA polymerase of the present invention may also be used to amplify DNA, e.g., by the procedure disclosed in U.S. Patent Nos. 4,683,195, 4,683,202 and 4,800,159.

The following examples are given to illustrate embodiments of the present invention as it is presently preferred to practice. It will be understood that the examples are illustrative, and that the invention is not to be considered as restricted except as indicated in the appended claims.

### EXAMPLE I

### PURIFICATION OF A THERMOSTABLE DNA POLYMERASE FROM PYROCOCCUS SPECIES

*Pyrococcus sp.* strain GB-D (ATCC No. 55239) was grown in the media described by Belkin, et al., *supra,* containing 10 g/l of elemental sulfur in 8 one liter bottles at 94°C for two days. The cells were cooled to room temperature, separated from unused sulfur by decanting and collected by centrifugation and stored at -70°C. The yield of cells was 1.4 g per liter.

11.5 g of cells obtained as described above, were suspended in 28 ml of buffer A (10 mM KP04 buffer, pH 7.4; 0.1 mM EDTA, 1.0 mM beta-mercaptoethanol) containing 0.1 M NaCl and sonicated for 5 minutes at 4°C. The lysate was centrifuged at 15,000 g for 30 minutes at 4°C. The supernatant solution was passed through a 18 ml Affigel blue column (Biorad). The column was then washed with 50 ml of buffer A containing 0.1 M NaCl. The column was eluted with a 300 ml linear gradient from 0.1 to 2.0 M NaCl in buffer A. The DNA polymerase eluted as a single peak at approximately 1.3 M NaCl and represented 90% of the activity applied. The peak activity of DNA polymerase (25 ml) was dialyzed against 1 liter of buffer A containing 100 mM NaCl, and then applied to 15 ml Phosphocellulose column, equilibrated with buffer A containing 100 mM NaCl. The column was washed with 50 ml of buffer A containing 100 mM NaCl, and the enzyme activity was eluted with 200 ml linear gradient of 0.1 to 1.0 M NaCl in buffer A. The activity eluted as a single peak at 0.6 M NaCl and represented 70% of the activity applied. The pooled activity (42 ml) was dialyzed against 500 ml of buffer A and applied to a 25 ml DEAE column. The column was washed with 50 ml of buffer A containing 0.1 M NaCl, and two-thirds of the enzyme activity passed through the column. The active fractions were pooled (30 ml) and applied to an 1.0 ml HPLC mono-S column (Pharmacia) and eluted with a 100 ml linear gradient in buffer A from 0.05 to 1.0 M NaCl. The activity eluted as a single peak at 0.22 M NaCl and represented 80% of the activity applied.

Purified *Pyrococcus sp.* polymerase was electrophoresed in SDS 10-20% polyacrylamide gel and stained with either Coomassie Blue or the colloidal stain (ISS Problue) previously described to detect protein. A faintly staining protein band was seen at about 92,000 to 97,000 daltons; this molecular weight determination was obtained by comparison on the same gel to the migration of the following marker proteins (Bethesda Research Laboratories): myosin, 200,000 daltons; phosphorylase B, 97,400 daltons; BSA, 68,000 daltons; ovalbumin, 43,000 daltons, carbonic anhydrase 29,000 daltons; b-lactoglobulin, 18,400 daltons; lysoyzme 14,300 daltons.

### EXAMPLE II

### CLONING OF PYROCOCCUS SPECIES DNA POLYMERASE GENE

Cross hybridization of *a Pyrococcus* genomic DNA library using radioactive probes prepared from the DNA polymerase gene of *Thermococcus litoralis* allowed for the identification and isolation of a DNA encoding the *Pyrococcus* DNA polymerase. This was accomplished as set forth below.

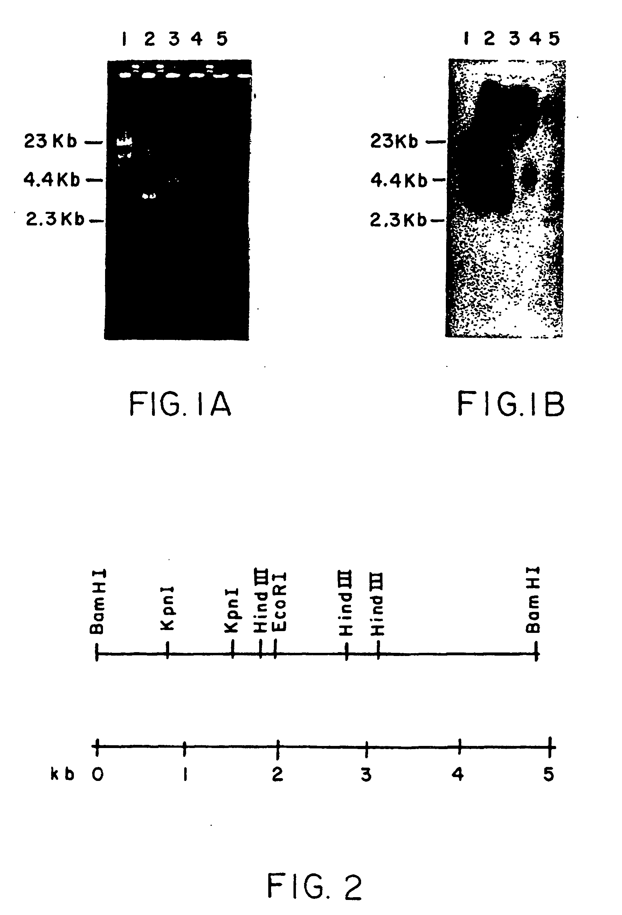

In order to determine which restriction enzymes would be most useful in preparation of the *Pyrococcus* genomic library, *Pyrococcus sp.* DNA was cut to completion with Eco RI, BamHI and HindIII. This DNA was subject to agarose gel electrophoresis (Figure 1A) and Southern hybridization (Figure 1B) using a DNA probe prepared as follows. A reaction mixture containing approximately 1 µg of the first EcoRI fragment of the *T. litoralis* DNA polymerase gene (bp 1-1274, obtainable from bacteriophage NEB#618, ATCC No. 40794) as a template in a commercial random priming kit (New England Biolabs, Inc.) was incubated for 1 hour at 37°C to produce a DNA probe of high specific activity. The probe was hybridized to *Pyrococcus sp.* DNA prepared above under moderately stringent conditions (Hybridization: overnight at 50°C, 4X SET, 0.1M sodium phosphate, pH 7, 0.1% Na pyrophosphate, 0.1% SDS, 1X Denhardts solution; Wash Conditions: wash 3X 20-30 min. 45°C, 0.1X SET, 0.1M sodium phosphate, pH 7, 0.1% Na pyrophosphate, 0.1% SDS. Maniatis, et al., *supra).* A single major band at about 5 Kb was detected in BamH I cut *Pyrococcus* DNA. EcoR I and Hind III gave multiple bands with this probe, indicating that these enzymes cut within the *Pyrococcus* polymerase gene.

Based on these results, a BamHI genomic library was constructed using the phage vector λDASH (Stratagene). Partial and complete BamHI digests of *Pyrococcus* DNA were prepared. A mixture of the partial and completely BamHI digested DNA was ligated into the BamHI site of λDASH. The ligation mixture was packaged using Gigapack Gold (Stratagene) according to manufacturer's instructions and plated on *E. coli* ER1458. The packaged phage library contained 1 x 10⁶ phage per ml.

³²P-labelled DNA probes of 3 fragments (SEQ ID NO:2, bp 1-1274, 1656-2660, and 3069-3737) of the *T. litoralis* DNA polymerase gene (obtainable from NEB#619, ATCC No. 40795) were prepared using a random primer kit (New England Biolabs, Inc.). The probes were used according to the method Benton & Davis (Maniatis, et al. *supra*) to screen the *Pyrococcus* genomic library using the hybridization conditions discussed above. About one per cent of the plaques were positive and ten positive plaques were picked and purified by reinfection and replating 3 times (until 90-100% of the plaques were positive for each isolate). Plate lysates (Maniatis, *supra*) of phage were prepared from each isolate and used to infect *E. coli* cultures. 0.1 ml of each plate lysate was mixed with 0.2 ml of cells (OD 600:2) The bacterial cells were harvested just before lysis and suspended in 0.05 M NaCl, 0.01M Tris (pH8.0), 0.1 mM EDTA, 0.1% Triton X-100 and 200 µg/ml lysozyme (3 volumes per volume of cells) and heated to 37°C for about 1 minute or until cell lysis occurred. The lysed extracts were immediately heated at 75°C for 30 minutes, centrifuged and the supernatant solution assayed for heat stable DNA polymerase activity, according to the method described above. Three of the ten isolates showed significant polymerase activity and the clone (B9) showing the most activity was investigated further.

The phage DNA was isolated from B9 and the insert DNA was examined by restriction enzyme digestion. Digestion with Sal I gave the expected two arms of λDASH plus a 15 Kb insert. Digestion with BamH I gave the two arms of λDASH plus three insert fragments of 7, 4.8 and 3 Kb. Each of these fragments were purified by agarose gel electrophoresis, eluted and ligated into the BamH I site of pUC19. The ligation mixtures were used to transform *E. coli* ER2207 which gives white colonies when plasmids contain an insert and blue colonies with no inserts on indicator agar media (X-gal plus IPTG). No white colony transformants were obtained with the 7 kb fragment. Three whites colonies and twenty-seven blue colonies were obtained with the 4.8 Kb fragment and twenty white and twenty-one blue colony transformants were obtained with the 3 Kb fragment. All three 4.8 Kb white colony transformants expressed heat stable DNA polymerase activity. None of the transformants with the 3 Kb fragment expressed heat stable polymerase activity. The three clones carrying the 4.8 Kb *Pyrococcus* DNA fragment all had about the same specific activity for heat stable DNA polymerase and one was picked for further study (NEB#720). This clone designated NEB#720 was deposited with the American Type Culture Collection, 12301 Parklawn Drive, Rockville, Maryland, under the terms of the Budapest Treaty, on October 1, 1991 and bears ATCC No. 68723. NEB#720 yielded 1700 units of DNA polymerase activity per gram of cells and was used for the large scale preparation of this enzyme.

A restriction endonuclease map of the 4.8 Kb BamH I fragment containing the *Pyrococcus sp.* DNA polymerase gene is shown in Figure 2. A partial DNA sequence of this gene is set forth in the Sequence Listing as SEQ ID NO:1. By comparison of the *Pyrococcus species* polymerase DNA and protein sequences of the present invention with *Thermococcus litoralis* DNA polymerase DNA and protein it was discovered that at least one intervening sequence is present within the *Pyrococcus* DNA polymerase gene. The *Pyrococcus* intervening sequence is within the conserved pol α motif region III, and begins at nucleotide 1839 of SEQ ID NO:1.

### EXAMPLE III

### PURIFICATION OF RECOMBINANT PYROCOCCUS SPECIES DNA POLYMERASE

*E. coli* NEB#720 (ATCC No. 68723) was grown in a 25 liter fermentor in media containing 10 g/liter tryptone, 5 g/liter yeast extract, 5 g/liter NaCl and 100 mg/liter ampicillin at 37°C and induced with 0.3 mM IPTG at mid exponential growth phase and incubated an additional 4 hours. The cells are harvested by centrifugation and stored at -70°C.

92 grams of cells were thawed and suspended in Buffer A (100 mM NaCl, 10 mM KPO₄ at pH 7.4, 0.1 mM EDTA, 0.1% Triton X-100 200 µg/ml lysozyme) to a total volume of 350 ml. The cells were lysed by incubation at 37°C until the mixture became extremely viscous (about 5 min.). The crude extract was immediately heated to 75°C for 30 minutes. The denatured *E*. *coli* proteins were removed by centrifugation and the supernatant solution was used for the isolation of the *Pyrococcus sp.* heat stable DNA polymerase.

The supernatant solution was brought to 0.3M NaCl and passed through a DEAE-cellulose column 2x30cm prepared in a buchner funnel to remove nucleic acids and the flow through passed through an Affi-Gel Blue chromatography column 4x10cm (125 ml) and washed with 300 ml 0.3M NaCl, 0.01M KPO₄ (pH 7.4) 0.1mM EDTA and then eluted with a 1500 ml linear gradient of NaCl from 0.3M to 2.0M in the same buffer.

The column fractions were assayed for DNA polymerase activity. Briefly, 1-4 µl of fractions were incubated for 5-10 minutes at 75°C in 50 µl of DNA polymerase buffer (10 mM KCl, 20 mM Tris-HCl (pH 8.8 at 24°C), 10 mM (NH₄)₂SO₄,2 mM MgSO₄ and 0.1% Triton X-100) containing 30 µM each dNTP and ³H-labelled TTP, 0.5 mg/ml activated calf thymus DNA and 100 µg/ml acetylated BSA. The assay mixtures were applied to Whatman 3 mm filters and the filters were subjected to three washes of 10% TCA followed by two washes of cold ethanol. After drying of the filters, bound radioactivity representing incorporation of ³H-TTP into the DNA was measured. The active fractions were pooled dialyzed against 0.1M NaCl, 0.01M KPO₄ (pH7.4), 0.1mM EDTA and then passed through a phosphocellulose column 4x12cm (150 ml) and the column washed with 300 ml of the same buffer then eluted with a linear gradient of NaCl from 0.1M to 1.5M. Active fractions were pooled and diluted with an equal volume of H₂O and passed through a 1.0 ml Pharmacia HPLC Mono Q column and eluted with a 60 ml linear gradient of NaCl from 0.05M to 1.0M. Active fractions were pooled and stored at -20°C. The *Pyrococcus sp.* DNA polymerase was approximately 50% pure at this stage as determined by visual assessment of a Coomassie Blue stained gel and had a specific activity for DNA synthesis of 70,000-100,000 units per mg, and represented 8% of enzyme activity present in the crude extracts.
The purified polymerase was substantially free of contaminating DNA and contaminating nucleases.

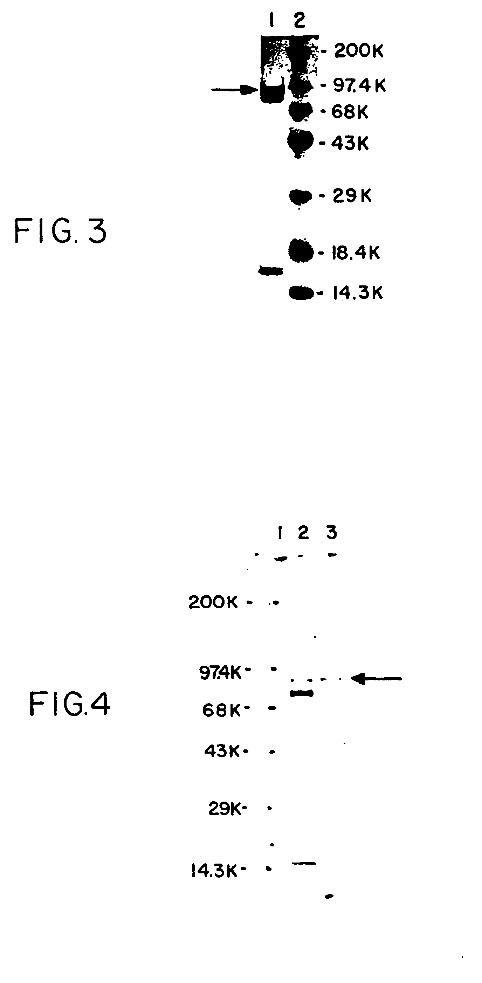

The DNA polymerase activity corresponded to major 92-97,000 Kd Coomassie Blue staining band in Figure 3. This was determined in the following manner. After electrophoresis in SDS 10-20% polyacrylamide gel and before staining with Coomassie Blue, the gel was soaked in 1.0% Triton X-100, 0.01M Tris HCl (pH7.4) overnight at 4°C with 3 changes of 200 ml each to remove sodium dodecylsulfate (SDS) from the gel and replace it with the non-ionic detergent Triton X-100. After the last wash, the gel was soaked in 0.1% Triton X-100, 0.01M Tris HCl (pH7.4) for 2 hours and then dried briefly with paper towel and placed on a glass plate. The gel was covered with a piece of Whatman No. 54 filter paper and 0.3 ml of assay buffer containing ³²P-d CTP (10-20x10⁶ cpm per µmole) added to the center of the filter paper. After the liquid had diffused towards the sides of the paper, a second glass plate was added on top of the filter paper and the glass-gel-paper-glass sandwich tightly taped together and incubated at 75°C for 60 minutes. The paper was removed from the gel, washed three times (30 minutes each wash) with 10% TCA containing 1mM dCTP and twice with isopropanol. The paper was air dried and exposed to an X-ray film overnight. When developed, a black spot on the film could be superimposed on the 92-97,000 Kd Coomassie Blue band on the stained gel indicating that the major band on the gel in Figure 3 is the DNA polymerase.

Western blot analysis (Towbin, et al, *PNAS* (1979) 76:4350-4354) of the purified recombinant protein using antibody prepared against the closely related DNA polymerase from *Thermococcus litoralis*, indicated a major band at 92,000-97,000 Kd identical to the Coomassie Blue stained band and at approximately the same location on the gel (Figure 4).

### EXAMPLE IV

### DETERMINATION OF 3'-5' PROOFREADING ACTIVITY

### 1. Response of T. Litoralis DNA Polymerase to the Absence of Presence of Deoxynucleotides

The levels of exonuclease activities associated with polymerases show very different responses to deoxynucleotides. Nonproofreading 5'-3' exonucleases are stimulated tenfold or greater by concomitant polymerization afforded by the presence of deoxynucleotides, while proofreading 3'-5' exonucleases are inhibited completely by concomitant polymerization. Lehman, I.R. *ARB* (1967) 36:645.

The *Pyrococcus sp.* DNA polymerase or polymerases with well-characterized exonuclease functions (T4 Polymerase, Klenow fragment) were incubated with 1 µg ³H-thymidine-labelled double-stranded DNA (10⁵ CPM/µg) in polymerization buffer (70 mM tris pH 8.8 at 24°C), 2 mM MgCl₂, 0.1% Triton and 100 µg/ml bovine serum albumin). After an incubation period of three hours (experiment 1) or four hours (experiment 2) at either 70°C (thermophilic polymerases) or 37°C (mesophilic polymerases), the exonuclease-hydrolyzed bases were quantified by measuring the acid-soluble radioactively-labelled bases.

As shown in Table 1, the Taq DNA polymerase, with its 5'-3' exonuclease activity, shows stimulation of exonuclease activity when deoxynucleotides were present at 30 µM. However, polymerases with 3'-5' proofreading exonuclease activities, such as the T4 polymerase, Klenow fragment of *E. coli* polymerase I, *T. litoralis* DNA polymerase or the *Pyrococcus* DNA polymerase showed the reverse, an inhibitory response to the presence of deoxynucleotides.

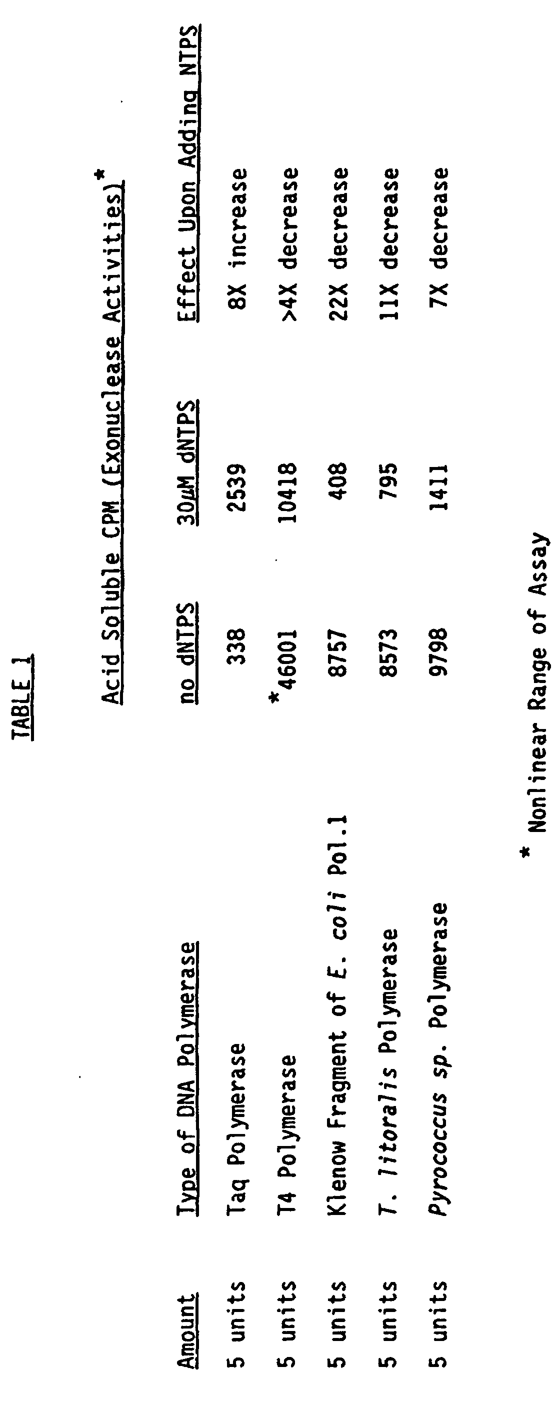

### 2. Comparison of 3'-5' Proofreading Activity of Pyrococcus sp. DNA Polymerase to Thermococcus litoralis DNA Polymerase

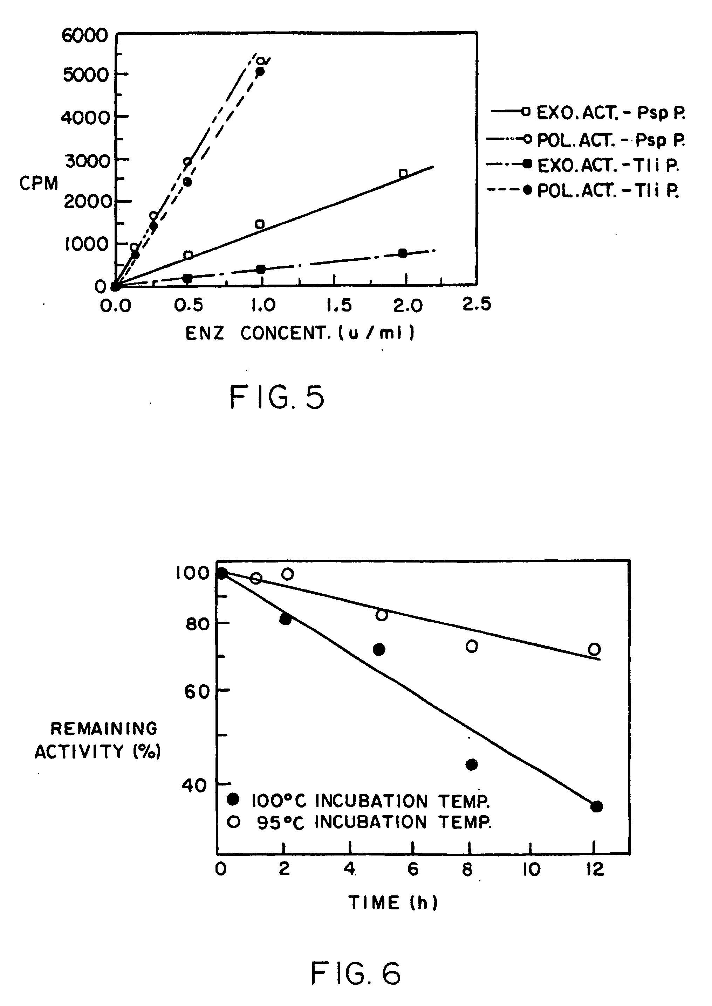

To further characterize the 3'-5' exonuclease activity of *Pyrococcus sp.* DNA polymerase, both enzymes were compared at the same DNA polymerization units for 3'-5' proofreading exonuclease activity (Figure 5). The results indicate that at equal DNA polymerization units, *Pyrococcus sp.* DNA polymerase has 2.5 times the exonuclease activity as *Thermococcus litoralis* DNA polymerase. This would suggest greater fidelity during DNA synthesis.

### EXAMPLE V

### PYROCOCCUS SP. DNA POLYMERASE HALF LIFE DETERMINATION

The thermostability or half-life of the *Pyrococcus sp.* DNA polymerase purified as described above in Example III was determined by the following method. Purified recombinant *Pyrococcus sp.* DNA polymerase (40 units/ml) was preincubated at 95°C or 100°C in reaction buffer lacking dNTPs and DNA (reaction buffer 10 mM KCl, 10 mM (NH₄)₂ SO₄, 20 mM Tris-HCl (pH 8.8 at 25°C), 2 mM MgSO₄, 0.1% TRITON X-100, supplemented with 0.1 mg/ml BSA). At times indicated in Figure 6, an aliquot of the enzyme mixture was diluted four-fold into reaction buffer at 70°C which contained [³H] labelled dNTPs and primed M13 DNA substrate (final concentration 0.2 mM and 20nM, respectively) and the initial rate of [³H] incorporation into acid insoluble material was monitored. Activity is expressed relative to activity present prior to treatment at 95°C or 100°C.

As shown in Figure 6, the half life of the *Pyrococcus sp.* DNA polymerase at 95°C was 23 hours, and 8 hours at 100°C.

## Claims

1. A purified thermostable enzyme obtainable from *Pyrococcus sp.* which catalyzes the polymerization of DNA, wherein said enzyme is obtainable from *E.coli* NEB#720 (ATCC # 68723).

2. The thermostable enzyme of claim 1, having a molecular weight of about 92,000 to 97,000 daltons.

3. The thermostable enzyme of claim 1, having a 3'-5' exonuclease activity.

4. The thermostable enzyme of claim 3, wherein the 3'-5' exonuclease activity is inactivated.

5. The thermostable enzyme of claim 1, having a half life of about 8 hours at 100°C.

6. The thermostable enzyme of claim 1, having a half life of about 23 hours at 95°C.

7. An isolated DNA sequence coding for the thermostable enzyme of claim 1.

8. A vector containing the DNA sequence of claim 7.

9. A microbial host transformed with the vector of claim 8.

10. The transformant of claim 9, wherein said transformant is *E*. *coli* NEB#720 (ATCC No. 68723).

11. A process for the preparation of *Pyrococcus sp.* DNA polymerase comprising culturing the transformed microbial host of any of the claims 9 or 10 under conditions suitable for the expression of *Pyrococcus sp.* DNA polymerase and recovering *Pyrococcus sp.* DNA polymerase.

12. The DNA of claim 7, wherein the DNA contains an intervening DNA sequence.

13. A process according to claim 11 for increasing expression of *Pyrococcus sp.* DNA polymerase comprising:
(a) deleting an intervening DNA sequence from DNA according to claim 7;
(b) introducing the DNA of step (a) into an expression vector;
(c) transforming a microbial host with the expression vector of step (b);
(d) culturing the transformed host of step (c), under conditions suitable for the expression of *Pyrococcus sp.* DNA polymerase; and
(e) recovering the polymerase.

## Patentansprüche

1. Gereinigtes thermostabiles Enzym, erhältlich von *Pyrococcus sp.*, das die Polymerisation von DNA katalysiert, wobei das genannte Enzym von *E. coli* NEB Nr. 720 (ATCC Nr. 68723) erhältlich ist.

2. Thermostabiles Enzym nach Anspruch 1, mit einer relativen Molekülmasse von etwa 92.000 bis 97.000 Dalton.

3. Thermostabiles Enzym nach Anspruch 1, mit einer 3'-5'-Exonucleaseaktivität.

4. Thermostabiles Enzym nach Anspruch 3, wobei die 3'-5'-Exonucleaseaktivität inaktiviert ist.

5. Thermostabiles Enzym nach Anspruch 1, mit einer Halbwertszeit von etwa 8 Stunden bei 100°C.

6. Thermostabiles Enzym nach Anspruch 1, mit einer Halbwertszeit von etwa 23 Stunden bei 95°C.

7. Isolierte DNA-Sequenz, die das thermostabile Enzym aus Anspruch 1 kodiert.

8. Vektor, der die DNA-Sequenz von Anspruch 7 enthält.

9. Mikrobieller Wirt, transformiert mit dem Vektor aus Anspruch 8.

10. Transformante nach Anspruch 9, wobei die genannte Transformante *E. coli* NEB Nr. 720 (ATCC Nr. 68723) ist.

11. Verfahren zur Herstellung von *Pyrococcus sp.* DNA-Polymerase, umfassend das Kultivieren des transformierten mikrobiellen Wirts nach einem der Ansprüche 9 oder 10 unter Bedingungen, die für die Expression von *Pyrococcus sp.* DNA-Polymerase und Wiedergewinnung von *Pyrococcus sp.* DNA-Polymerase geeignet sind.

12. DNA nach Anspruch 7, wobei die DNA eine Intervening-DNA-Sequenz enthält.

13. Verfahren nach Anspruch 11 für die Steigerung der Expression von *Pyrococcus sp.* DNA-Polymerase, umfassend die folgenden Schritte:
(a) Deletieren einer Intervening-DNA-Sequenz von DNA nach Anspruch 7;
(b) Einleiten der DNA aus Schritt (a) in einen Expressionsvektor;
(c) Transformieren eines mikrobiellen Wirts mit dem Expressionsvektor von Schritt (b);
(d) Kultivieren des transformierten Wirts aus Schritt (c) unter Bedingungen, die für die Expression von *Pyrococcus sp.* DNA-Polymerase geeignet sind; und
(e) Wiedergewinnen der Polymerase.

## Revendications

1. Enzyme thermostable purifiée pouvant être obtenue de *Pyrococcus sp.* qui catalyse la polymérisation de l'ADN, où ladite enzyme peut être obtenue d'*E*. *coli* NEB#720 (ATCC # 68723).

2. Enzyme thermostable de la revendication 1, ayant un poids moléculaire d'environ 92.000 à 97.000 daltons.

3. Enzyme thermostable de la revendication 1, ayant une activité exonucléase 3'-5'.

4. Enzyme thermostable de la revendication 3, dans laquelle l'activité exonucléase 3'-5' est inactivée.

5. Enzyme thermostable de la revendication 1, ayant une demi-vie d'environ 8 heures à 100°C.

6. Enzyme thermostable de la revendication 1, ayant une demi-vie d'environ 23 heures à 95°C.

7. Séquence d'ADN isolée codant l'enzyme thermostable de la revendication 1.

8. Vecteur contenant la séquence d'ADN de la revendication 7.

9. Hôte microbien transformé avec le vecteur de la revendication 8.

10. Le transformant de la revendication 9, où ledit transformant est *E. coli* NEB#720 (ATCC n° 68723).

11. Procédé pour la préparation d'ADN polymérase *Pyrococcus sp.* comprenant cultiver l'hôte microbien transformé de l'une quelconque des revendications 9 ou 10 dans des conditions qui conviennent à l'expression de l'ADN polymérase *Pyrococcus sp.* et récupérer l'ADN polymérase *Pyrococcus sp.*

12. L'ADN de la revendication 7, où l'ADN contient une séquence d'ADN interposée.

13. Procédé selon la revendication 11 pour augmenter l'expression de l'ADN polymérase *Pyrococcus sp.* comprenant :
(a) éliminer une séquence d'ADN interposée de l'ADN selon la revendication 7;
(b) introduire l'ADN de l'étape (a) dans un vecteur d'expression;
(c) transformer un hôte microbien avec le vecteur d'expression de l'étape (b);
(d) cultiver l'hôte transformé de l'étape (c), dans des conditions qui conviennent à l'expression de l'ADN polymérase *Pyrococcus sp.;* et
(e) récupérer la polymérase.
